# EUROPÄISCHE PATENTANMELDUNG

(11) **EP 0 553 721 A1**
(43) Veröffentlichungstag der Anmeldung: **04.08.1993**
(21) Anmeldenummer: 93100938.5
(22) Anmeldetag: 22.01.1993
(51) Int. Cl.: C07D 307/08

(54) **Verfahren zur Trennung eines Gemisches aus Tetrahydrofuran und Wasser in seine Bestandteile**

(30) Priorität: 30.01.1992 DE 4202564
(71) Anmelder: BASF Aktiengesellschaft, D-67063 Ludwigshafen (DE)
(72) Erfinder: Krug, Joseph, Dr., W-6719 Weisenheim am Berg (DE); Palm, Christof, Dr., W-6700 Ludwigshafen (DE); Broellos, Klaus, Dr., W-6104 Seeheim-Jugenheim (DE); Wolf, Dieter, Dr., W-6718 Grünstadt (DE)

(57) **Zusammenfassung**

Verfahren zur Trennung eines Gemisches aus Tetrahydrofuran und Wasser in seine Bestandteile, wobei die Trennung mittels Pervaporation durchgeführt wird.

## Beschreibung

Die Erfindung betrifft ein Verfahren zur Trennung eines Gemisches aus Tetrahydrofuran und Wasser in seine Bestandteile.

Tetrahydrofuran ist ein häufig verwendetes Lösungsmittel, Reaktionsmedium und Einsatzprodukt für verschiedene Synthesen in der chemischen Industrie, beispielsweise bei der Herstellung von Klebstoffen, Speziallacken, Beschichtungen, bei der Extraktion spezieller Wirkstoffe, für die Umkristallisation bestimmter Verbindungen oder als Ausgangsmaterial für verschiedene Synthesen in einer Reihe von Reaktionen. Nach dem Einsatz fällt dabei das Tetrahydrofuran oft wasserhaltig an.

Da ein azeotropes Gemisch in nur einer stationär arbeitenden Kolonne nicht vollständig in seine Komponenten zerlegt werden kann, ist eine Auftrennung dieses Gemisches, beispielsweise durch einfache Rektifikation nur bis zum Azeotrop möglich. Das Azeotrop liegt bei 94,7 Gew.-% Tetrahydrofuran und 5,3 Gew.-% Wasser bei einem Druck von 1013 mbar und siedet bei 64°C. (Vergleiche Horsley, L.H., "Azeotropic Data III" Advances in Chemistry, Series 116, 1973, ACS, Washington D.C., USA.)

Daher werden in der Technik oft destillative Sonderverfahren eingesetzt, die entweder durch die Ausnutzung systemimmanenter Eigenschaften, wie beispielsweise dem Druckwechselverfahren, oder durch die Zugabe eines Hilfsstoffes, wie beispielsweise der Schleppmitteldestillation, diese Destillationsgrenze überwinden. (Vergleiche Destillation und Rektifikation, in Ullmanns Encyklopädie der technischen Chemie, 4. Auflage, Band 2, Seiten 489 bis 545.)

Alle diese Sonderverfahren sind jedoch aufwendig, da sie mindestens zwei und häufig mehrere Kolonnen und sonstige Apparate wie Druckerhöhungsstufen, Dekanter usw. und die damit verbundenen Verschaltungen, Rückführungen, Hilfsstoffaufarbeitungen usw. benötigen.

Der Erfindung liegt daher die Aufgabe zugrunde, wasserhaltiges Tetrahydrofuran einfach und ohne große Verluste auf Reinheiten größer 99,9 Gew.-% Tetrahydrofuran zu entwässern.

Diese Aufgabe wird erfindungsgemäß dadurch gelöst, daß die Trennung mittels Pervaporation durchgeführt wird, wobei das Gemisch flüssig oder gasförmig (Dampfpermeation) über eine hydrophile Membrane geführt wird.

Das Verfahren kann technisch sowohl in Batch- als auch in kontinuierlicher Fahrweise betrieben werden. Beide Verfahrensmöglichkeiten werden nachstehend detailliert beschrieben.

### Beispiel 1

Batchfahrweise, Entwässerung von Tetrahydrofuran durch Pervaporation.

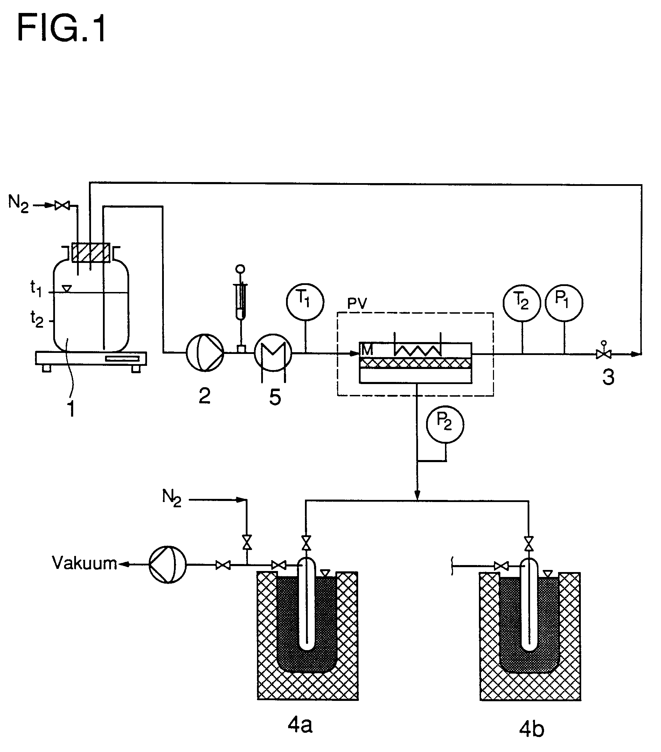

Das diskontinuierlich durchgeführte Verfahren ist in Figur 1 dargestellt. Dabei wird ein Gemisch aus 90 Gew.-% Tetrahydrofuran und 10 Gew.-% Wasser mit 1149 g im Behälter 1 bei Raumtemperatur vorgelegt. Diese Vorlaufmenge wird über die Pumpe 2 und die Testzelle PV mit der Membranfläche M von 100 cm² ca. 7 mal in der Stunde im Kreis gefahren. Am Druckhalteventil 3 wird ein Überdruck von 2,1 bar eingestellt. Auf der Permeatseite liegt ein absoluter Druck von 15 mbar an. Das Permeat wird in den Kühlfallen 4a und 4b bei ca. -70°C ausgefroren. Die Analyse des Permeats und des Behälterinhalts erfolgt in den ersten 6 Stunden stündlich, danach im Durchschnitt alle 6 Stunden. Nach der An-Einfahrweise wird das Feed vom Thermostaten 5 innerhalb einer Stunde von Raumtemperatur auf ca. 80°C erwärmt und konstant gehalten (Zeitpunkg t1). Der Temperaturunterschied zwischen der Feedtemperatur T1 (PV-Ein) und der Retentattemperatur T2 (PV-Aus) beträgt dann zum Zeitpunkt t1 ca. 2,1°C, zum Zeitpunkt t2 ca. 0,1°C und im Mittel ca. 1°C. Nach 48 Stunden (Zeitpunkt t2) ergibt sich eine Endproduktmenge von 1029 g mit 99,9 Gew.-% Tetrahydrofuran und ca. 1000 Gew.-ppm Wasser. Durch die hydrophile Membran sind 120 g mit 94,9 Gew.-% Wasser und 5,1 Gew.-% Tetrahydrofuran permeiert. Der Fluß hat dabei von 1,5 kg/(m²h) auf ca. 0,07 kg/(m²h) abgenommen.

### Beispiel 2

Kontinuierliche Fahrweise, Entwässerung von Tetrahydrofuran durch Pervaporation.

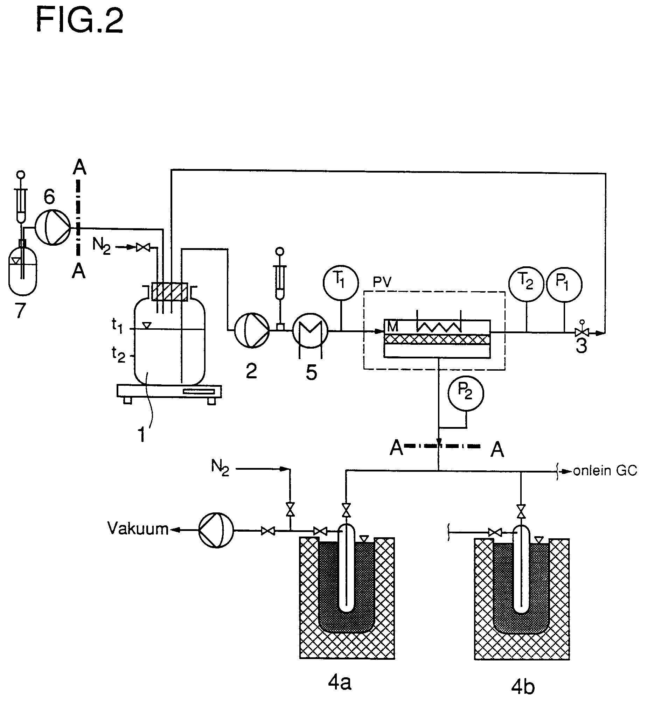

Das kontinuierlich durchgeführte Verfahren ist in der Figur 2 dargestellt. Dabei wird ein Gemisch aus 99,93 Gew.-% Tetrahydrofuran und 700 Gew.-ppm Wasser mit ca. 2 kg im Behälter 1 bei Raumtemperatur vorgelegt. Die Pumpe 2 fördert ca. 6,5 kg/h im Kreis. Die Membranfläche M der Pervaporation beträgt 100 cm². Am Druckhalteventil 3 wird ein Überdruck von 2,9 bar eingestellt. Auf der Permeatseite liegt ein absoluter Druck von 15 mbar an. Das Permeat wird etnweder in den Kühlfallen 4a oder 4b bei ca. -80°C ausgefroren oder im GC-Onlinebetrieb analysiert. Nach der Anfahrweise wird das Feed vom Thermostaten 5 innerhalb einer Stunde von Raumtemperatur auf ca. 90°C erwärmt und konstant gehalten. Der Temperaturunterschied zwischen der Feedtemperatur T1 (PV-Ein) und der Retentattemperatur T2 (PV-Aus) beträgt dann ca. 0,2°C. Über eine Dosierpumpe 6 wird aus der Vorlage 7 dem Behälter 1 ungefähr soviel Gemisch zugeführt, wie Permeat bei der Pervaporation anfällt (Schnitt A-A). Die Schwankungsbreite in der Feed-Retentatzusammensetzung beträgt dabei ca. 200 Gew.-ppm Wasser. Das Permeat besteht aus ca. 97 Gew.-% Wasser. Der Fluß beträgt ca. 0,04 kg/(m²h). Die Einfahrzeit der Membran beträgt 6 Tage und diese stationäre Betriebsweise mit konstanter Feedzusammensetzung wurde im Anschluß daran über einen Zeitraum von ca. 24 Stunden gefahren.

Die mit der Erfindung erzielten Vorteile bestehen in einer einfacheren und damit wirtschaftlicheren Verfahrensführung bei der Entwässerung von Tetrahydrofuran über das Azeotrop mittels Pervaporation auf Reinheiten größer 99,9 Gew.-% Tetrahydrofuran.

## Patentansprüche

1. Verfahren zur Trennung eines Gemisches aus Tetrahydrofuran und Wasser in seine Bestandteile, dadurch gekennzeichnet, daß die Trennung mittels Pervaporation durchgeführt wird.

2. Verfahren gemäß Anspruch 1, dadurch gekennzeichnet, daß die Trennung mittels einer hydrophilen Membrane durchgeführt wird.
